# EUROPÄISCHE PATENTANMELDUNG

(11) **EP 1 306 360 A2**
(43) Veröffentlichungstag der Anmeldung: **02.05.2003**
(21) Anmeldenummer: 02018989.0
(22) Anmeldetag: 27.08.2002
(51) Int. Cl.: C07C 31/30

(54) **Alkalialkoholat- und Erdalkalialkoholat-Granulate**

(30) Priorität: 15.10.2001 DE 10150328
(71) Anmelder: Degussa AG, 40474 Düsseldorf (DE)
(72) Erfinder: Harthun, Andreas, Dr., 63776 Mömbris (DE); Theis, Christoph, Dr., 53859 Niederkassel (DE); Noppe, André, 53757 Sankt Augustin (DE); Metz, Josef, Dr., 45770 Marl (DE)

(57) **Zusammenfassung**

Die Erfindung betrifft Granulate von Alkali- und Erdalkali-Alkoholaten von aliphatischen, unverzweigten oder verzweigten, gesättigten oder ungesättigten Alkoholen, mit insbesondere 1 bis 6 Kohlenstoffatomen im alkoholischen Rest. Die Granulate bieten erhebliche handhabungstechnische Vorteile gegenüber den bisher üblichen Lösungen oder Pulvern.

## Beschreibung

Die Erfindung betrifft Alkali- und Erdalkali-Alkoholate in Granulat-Form.

Alkoholate werden für vielfältige Anwendungszwecke eingesetzt. Als Beispiele hierfür seien Aldol-Additionen, Veresterungen/Umesterungen, Malonester-Synthesen, Etherbildungen und der sonstige allgemeine Einsatz als Base genannt. Die Alkoholate finden weiterhin breite Anwendung in der Lebensmittelindustrie wie zum Beispiel bei der Margarineherstellung oder der Vitamin A-Synthese, in der Pharmachemie wie zum Beispiel bei der Herstellung von Antibiotika, Schmerzmitteln, Chemotherapeutika und Epileptika, in der Agrochemie wie zum Beispiel bei der Herstellung von Herbiziden und Fungiziden und in vielen weiteren Anwendungsbereichen wie zum Beispiel bei der Herstellung optischer Aufheller, UV-Absorber und Photoinitiatoren.
Die Alkoholate werden dazu in der Regel in Form ihrer alkoholischen Lösung oder, wenn der Alkohol unerwünscht und es technisch und logistisch möglich ist, bei gegebener Verfügbarkeit in Pulverform eingesetzt.

Nachteilig wirkt sich bei Einsatz eines Alkoholatpulvers die Feinstaubfreisetzung bei Ab- und Umfüllvorgängen aus, die aufgrund der stark ätzenden Wirkung der Alkoholate und der leichten Selbstentzündlichkeit an der Atmosphäre ein hohes Gefahrenpotential für Mensch und Maschine birgt.

Bei dem Einsatz von Alkoholaten als Reaktionspartner häufig auftretende und schwierig zu handhabende Exothermien stellen außerdem ein zusätzliches, nicht unerhebliches Verfahrensrisiko dar. Diese Gefahrenpotentiale erfordern sehr hohen verfahrenstechnischen und apparatetechnischen Aufwand.

Es bestand daher die Aufgabe, Alkali- und Erdalkali-Alkoholate in einer Form zur Verfügung zu stellen, die die geschilderten Nachteile, insbesondere also die ätzende Feinstaubentwicklung bei der Handhabung, nicht aufweist.
Es wurde gefunden, dass sich Alkali- und Erdalkali-Alkoholate von Alkoholen mit 1 bis 12, vorzugsweise 1 bis 6, Kohlenstoffatomen mit bestimmten verfahrenstechnischen Maßnahmen in eine feste Granulatform bringen lassen, durch die sich die Risiken beim Umgang mit den genannten Alkoholaten und deren Einsatz bei den üblichen Reaktionen erheblich reduzieren und minimieren lassen. Gegenstand der Erfindung sind daher Alkali- und Erdalkali-Alkoholate von Alkoholen mit 1 bis 12, vorzugsweise 1 bis 6, Kohlenstoffatomen in Granulatform vorzugsweise hergestellt nach dem Verfahren der Wirbelschicht-Sprühgranulation. Die Alkoholate in Granulatform sind als grobkörniges Material leicht zu lagern, fördern, dosieren und mischen, da sie in dieser Festtstoffform nicht stauben, nicht agglomerieren und nicht zur Brückenbildung neigen.

Als Alkali-Kationen können im Alkoholat Lithium-, Natrium- und/oder Kalium-Ionen enthalten sein. Bevorzugte Alkali-Alkoholate sind insbesondere Natrium- und Kalium-Alkoholate.
Als Erdalkali-Kationen können im Alkoholat Magnesium- und/oder Calcium-Ionen enthalten sein. Bevorzugte Erdalkali-Alkoholate sind insbesondere Magnesium-Alkoholate.

Der alkoholische Rest im Alkoholat entstammt einem aliphatischen, unverzweigten oder verzweigten, gesättigten oder ungesättigten Alkohol mit 1 bis 12, vorzugsweise mit 1 bis 6 , ganz besonders bevorzugt 1 bis 4 Kohlenstoffatomen.

Als Reste seien vorzugsweise die sich vom Methanol, Ethanol, n-Propanol, Isopropanol, Allylalkohol, n-Butanol, iso-Butanol, tert.-Butanol, Amylalkohol (Pentanol) einschließlich der verschiedenen Isomeren des Pentanols und Hexanol einschließlich der verschiedenen Isomeren des Hexanols ableitenden Reste genannt. Die Erfindung betrifft aber insbesondere die Granulate von Alkoholaten des Methanols, des Ethanols, des Propanols sowie des Butanols in den verschiedenen isomeren Formen. Die Auflistung ist jedoch lediglich beispielhaft und keinesfalls einschränkend zu interpretieren.

Der Alkoholatgehalt des Granulates beträgt 50 - 100 Gew.-%, vorzugsweise 90 - 99 Gew.-% und insbesondere > 98 Gew.-%.
Als verfahrenstechnische Maßnahme zur Herstellung der Granulate kann die Wirbelschicht-Sprühgranulation angewendet werden. Die Granulatform und der Granulatdurchmesser können durch die Herstell- und Trocknungsbedingungen eingestellt und variiert werden. Vorzugsweise liegt das Granulat in einer asymmetrischen, näherungsweise sphärischen Form vor.

Der Durchmesser der Granulate reicht vom Mikrometer bis in den Zentimeterbereich. Vorzugsweise werden Granulate mit einem Durchmesser von 100 µm bis 10 mm erzeugt. Da die Granulatgröße wesentlich von den Granulationsbedingungen abhängt, ist die Angabe der Granulatgröße nicht einschränkend zu interpretieren.

Durch die Granulatform wird der Fließfähigkeitsindex (ffc) gegenüber einem pulverförmigen Alkoholat stark erhöht, was zu erheblichen Handhabungsvorteilen führt.

Weiterhin wird die Auflösungsgeschwindigkeit unter anderem durch die Granulatgröße bestimmt und damit die gewünschte Reaktion vergleichmäßigt. Dadurch wird die Exothermie bei chemischen Umsetzungen wesentlich besser beherrschbar.

Die Selbstentzündungstemperatur (ohne Zeitbegrenzung) wird zwar nicht herabgesetzt, jedoch kann für das Granulat eine Staubexplosion ausgeschlossen werden, da bei Um- und Abfüllvorgängen nahezu kein Stauben auftritt.

Die breiteste Anwendung der festen Alkalialkoholate findet Natriummethanolat. Aus diesem Grund werden nachfolgend exemplarisch die Stoffeigenschaften von Natriummethanolat-Pulver und Natriummethanolat-Granulat vergleichend gegenübergestellt. Die Eigenschaftsunterschiede sind jedoch allgemein für die Alkoholatgranulate gemäß vorstehender Beschreibung und Anspruch 1 vorhanden und sollen keinesfalls ausschließlich für Natriummethanolat gelten.

| | Natriummethanolat-Pulver | Natriummethanolat-Granulat |
|---|---|---|
| Schüttdichte [g/l] | ca. 500 | ca. 400 |
| Stampfdichte [g/l] | ca. 645 | ca. 410 |
| Verdichtung [%] | 22,5 | 2,5 |
| Fließfähigeitsindex (ffc) | 5,19 | 18,74 |
| Spezifische Oberfläche [m²/g] (DIN | 6,4 | 17 |
| 66131) | | |
| Selbstentzündungstemperatur bei Luftzutritt [°C] | 55 - 60 | 50 - 55 |

Anhand der Stoffdaten ist ersichtlich, dass es sich bei dem Natriummethanolat-Granulat um einen Feststoff mit für die Handhabung wesentlich vorteilhafteren Eigenschaften im Vergleich zu dem Natriummethanolat-Pulver handelt. Die angegebene, geringere Schüttdichte des Granulates ist wesentlich vom Granulatdurchmesser abhängig, der wiederum durch Variation der Granulationsbedingungen verändert werden kann. Das untersuchte Granulat verfügt über einen durchschnittlichen Durchmesser von 2 mm. Diese Angaben haben jedoch lediglich beispielhaften Charakter und sollen keinesfalls den Granulatdurchmesser oder die technisch realisierbare Schüttdichte der Alkoholatgranulate einschränken.

Das Granulat besitzt erheblich bessere Fließeigenschaften als das Pulver und führt bei einem Stampftest lediglich zu einer Kompaktierung von 2,5 %. Demgegenüber verdichtet sich das Pulver um 22,5 %.

Erfindungsgemäß wird mit dem Alkoholatgranulat eine Darreichungsform für Alkoholatfeststoffe zur Verfügung gestellt, die über erheblich bessere Lager-, Förder- und Dosiereigenschaften verfügt als das entsprechende Pulver.

Die Lösungsgeschwindigkeit des Natriummethanolat-Feststoff in unterschiedlichen Alkoholen ist nachfolgend zusammengefasst. 25 g Feststoff werden hierzu unter Inertgasbedingungen in 100 g Ethanol und 40 g Feststoff in 100 g Methanol gerührt.

| | Methanol | Ethanol |
|---|---|---|
| Natriummethanolat-Pulver [min] | 6 | 10 |
| Natriummethanolat-Granulat [min] | 16 | 12 |

Der Vergleich der Lösegeschwindigkeiten von Natriummethanolat-Pulver und -Granulat verdeutlicht, dass sich das Granulat bei gleicher Rührgeschwindigkeit langsamer löst als das entsprechende Pulver. Bei diskontinuierlichen exothermen Reaktionen weist aber gerade diese Stoffeigenschaft des Granulates erfindungsgemäße Vorteile auf. Eine herabgesetzte Lösegeschwindigkeit des Alkoholates ermöglicht hierbei eine vergleichmäßigte Reaktionsführung der Umsetzung mit dem Alkoholat. Dieser Effekt ist sogar in polaren, protischen Lösemitteln zu beobachten, in denen sich Alkoholate nur in geringem Maße lösen.

Ein Beispiel hierfür ist die Carbonylierung von Propionsäureester in Dimethylformamid. Die Reaktion ist infolge der dabei auftretenden extremen CO-Aufnahme und Exothermie eine besonders gut geeignete Modell-Reaktion.

### Vergleichsbeispiel:

Carbonylierung von Propionsäureethylester mit Natriummethanolat-Pulver:
Die Carbonylierung von Propionsäureethylester mit Natriummethanolat-Pulver in Dimethylformamid zeigt ein Temperaturmaximum von 80 °C, wobei gleichzeitig ein Druckabfall im Autoklaven von ca. 10 bar bei gleichzeitiger Erhöhung des CO-Volumenstroms um 500 % auftritt.

### Beispiel:

Carbonylierung von Propionsäureethylester mit Natriummethanolat-Granulat:
Die Carbonylierung von Propionsäureethylester mit Natriummethanolat-Granulat verläuft wesentlich moderater. Die Maximaltemperatur beträgt lediglich 70 °C, bei einem nicht signifikanten Druckabfall und einem maximalen CO-Volumenstrom von 20 %, bezogen auf den maximalen CO-Volumenstrom des Vergleichsbeispiels.
Der gleichmäßige Reaktionsverlauf hat hierbei überraschenderweise keinen Einfluss auf die Raum-Zeit-Ausbeute.

Das Beispiel der Carbonylierung von Propionsäureethylester mit Natriummethanolat-Granulat verdeutlicht, dass das Alkoholatgranulat nicht nur mechanische Handhabungsvorteile besitzt, sondern auch erhebliche Vorteile bei der Reaktionsführung exothermer Reaktionen aufweist.

## Patentansprüche

1. Alkali- und Erdalkali-Alkoholat-Granulate.

2. Alkoholat-Granulate nach Anspruch 1,
dadurch gennzeichnet, dass
der Durchmesser des Granulates durch die Herstell- und Trocknungsbedingungen eingestellt wird.

3. Alkoholat-Granulate nachAnspruch 1,
**dadurch gekennzeichnet, dass**
der Alkoholteil von aliphatischen, gesättigten oder ungesättigten Alkoholen mit 1 bis 6 Kohlenstoffatomen stammt.

4. Alkoholat-Granulate nach Anspruch 3,
**dadurch gekennzeichnet, dass**
der Alkohol aus der Gruppe, die durch Methanol, Ethanol, Propanol, Isopropanol, Allylalkohol, Butanol, iso-Butanol, tert.-Butanol, Pentanol und Isomere des Pentanols und Hexanol und Isomere des Hexanols gebildet wird, ausgewählt wird.

5. Alkoholat-Granulate nach einem der vorhergehenden Ansprüche,
**dadurch gekennzeichnet, dass**
als Alkalikationen Natrium- oder Kalium-Ionen vorhanden sind.

6. Alkoholatgranulate nach einem der Ansprüche 1 bis 4,
**dadurch gekennzeichnet, dass**
als Erdalkalikationen Magnesium-Ionen vorhanden sind.

7. Alkoholat-Granulate nach einem der vorhergehenden Ansprüche,
**dadurch gekennzeichnet, dass**
die Granulate nach dem Verfahren der Wirbelschicht-Sprühgranulation hergestellt werden.

8. Alkoholat-Granulate nach einem der vorhergehenden Ansprüche,
**dadurch gekennzeichnet, dass**
der Alkoholat-Gehalt 50 - 100 Gew.-% beträgt.

9. Alkoholat-Granulate nach einem der vorhergehenden Ansprüche,
**dadurch gekennzeichnet, dass**
der Alkoholat-Gehalt > 98 Gew.-% beträgt.
